# EUROPÄISCHE PATENTANMELDUNG

(11) **EP 1 005 846 A2**
(43) Veröffentlichungstag der Anmeldung: **07.06.2000**
(21) Anmeldenummer: 99102568.5
(22) Anmeldetag: 10.02.1999
(51) Int. Cl.: A61F 13/14, A61F 5/30

(54) **Bandage, insbesondere Rückenbandage**

(30) Priorität: 01.12.1998 DE 29821392 U
(71) Anmelder: Schnittker, Franz-Joseph, Dr., 32825 Blomberg (DE)
(72) Erfinder: Schnittker, Franz-Joseph, Dr., 32825 Blomberg (DE)
(74) Vertreter: Eikel, Cordula

(57) **Zusammenfassung**

Eine Bandage, insbesondere Rückenbandage ist dadurch gekennzeichnet, daß sie wenigstens mit einer Einrichtung zur Koppelung wenigstens einer Pelotte versehen ist, so daß eine Bandage geschaffen wird, die sich durch hohe Flexibilität, d.h. verschiedenste Einsatzmöglichkeiten bei gleichzeitig geringen Kosten und damit hohem Wirkungsgrad auszeichnet, ohne daß es für die verschiedensten Anwendungsgebiete jeweils gesonderter Bandagen bedarf.

## Beschreibung

Die Erfindung betrifft eine Bandage, insbesondere Rückenbandage, mit den Merkmalen des Oberbegriffes des Schutzanspruchs 1.

Bandagen werden für die verschiedensten Zwecke benutzt, d.h. z.B. unter Einsatz verschiedener Pelotten, wie z.B. unter Einsatz von Sacralpelotten oder z.B. Lumbalpelotten oder thermoelastischer Pelotten, so daß verschiedenartige Bandagen je nach Verwendungszweck zum Einsatz gelangen.

Darüber hinaus ist es für andere Bereiche notwendig, z.B. Fixationsgurte für die Anwendung bei z.B. Wärmetherapien vorzusehen sowie ggfls. auch bei der Anwendung von z.B. Stimulationsgeräten unter Verwendung von Hautelektroden wenngleich diese auch gesondert auf die Haut, d.h. z.B. durch entsprechende Haftmittel aufgebracht werden können.

Daraus ergibt sich, daß eine Vielzahl von z.B. verschiedene Bandagen und/oder Fixationsgurten bereit gehalten werden müssen.

Die Aufgabe der Erfindung ist es daher, eine Bandage bereitzustellen, die sich durch hohe Flexibilität bei gleichzeitig geringen Kosten und damit hohem Wirkungsgrad auszeichnet.

Diese Aufgabe wird bei einer Bandage der eingangs genannten Gattung mit den Merkmalen des kennzeichnenden Teiles des Schutzanspruches 1 gelöst. Weitere vorteilhafte Ausgestaltungen der Erfindung sind Gegenstand der Unteransprüche.

Dadurch, daß eine Bandage, insbesondere Rückenbandage bereitgestellt wird, die mit wenigstens einer Einrichtung zur Koppelung wenigstens einer Pelotte versehen ist, wird eine Bandage geschaffen, die sich durch ein Modularsystem auszeichnet, daß den Einsatz der verschiedensten Pelotten mittels ein und derselben Bandage ermöglicht, ohne daß jeweils eine gesonderte Bandage bereitgestellt werden muß, d.h. die Bandage kann unabhängig von Form und Größe der jeweiligen Pelotte zum Einsatz gelangen.

Ist die Einrichtung eine Tasche zur Aufnahme wenigstens einer Pelotte, so ermöglicht dies eine einfachste Handhabung, ohne daß es dazu spezieller Kenntnisse bedarf.

Ist die Pelotte mit wenigstens einem Koppelungselement versehen und ist die Tasche mit wenigstens einem dazu komplementär bzw. kompatibel ausgestalteten Koppelungselement versehen, so ermöglicht dies eine einfache aber um so praktikablere Positionierung der Pelotte in der Tasche, ohne daß die Taschengröße der jeweiligen Pelotte angepaßt sein muß.

Ist die Einrichtung ein Haftverschluß und weist die Tasche und Pelotte miteinander in Wirkungseingriff brinbare Elemente des Haftverschlusses auf, so kann dadurch auf einfachste aber um so praktikablere Weise eine Positionierung und Depositionierung der Pelotte vorgenommen werden.

Ist die Bandage mit wenigstens einer Einrichtung zur Positionierung und Fixierung von wenigstens einer Elektrode, insbesondere Hautelektrode, versehen, so ermöglicht dies, eine Koppelung der Bandage mit z.B. einem Stimulationsgerät, um an die betroffenen, schmerzenden Zonen elektrische Impulse weiterzugeben und Schmerzreize zu unterdrücken bzw. vollständig zu beseitigen, d.h. die Bandage fungiert hier als Fixationsgurt mit TENS oder ITT (intermittierender Thermotherapie), d.h. im hier vorliegenden Beispiel auch als Träger der entsprechenden Elektroden und/oder Wirkelemente mit oder ohne Pelotte, d.h. die Bandage kann als reiner Fixationsgurt benutzt werden oder auch bei Bedarf in Kombination mit einer Pelotte.

Ist die Einrichtung wenigstens ein Element eines Haftverschlusses aufweisend ausgestaltet, daß mit wenigstens einem kompatibel bzw. komplementär dazu ausgestalteten Element angeordnet an der Elektrode in Wirkungseingriff bringbar ist, so ist dadurch die Möglichkeit einer Positionierung und Fixierung von Elektroden an der erfindungsgemäßen Bandage geschaffen, die sich durch ein einfaches aber um so wirkungsvolleres und praktikableres handling auszeichnet.

Ist eine Einrichtung zur Aufnahme einer Steuereinheit eines Stimulationsgerätes vorgesehen, so ermöglicht dies die Positionierung eines Steuergerätes auf einfachste Art und Weise und eröffnet damit dem Anwender die Möglichkeit des Anwendenden sich frei zu bewegen.

Ist wenigstens eine Einrichtung zur Positionierung und Fixierung eines elektronischen Wirkelementes, insbesondere zur Erzeugung wechselnder Temperaturphasen vorgesehen, so ermöglicht dies auch die Positionierung und damit Festlegung eines elektronischen Wirkelementes, das z.B. durch eine Andruckpelotte auf die schmerzenden Zonen fixiert werden kann, so daß über die elektronische Steuereinheit im Schmerzzentrum wechselnde Temperaturphasen erzeugt werden, ohne daß es hierzu gesonderter Vorrichtungen wie entsprechender Fixationsgurte bedarf.

Ist die Einrichtung wenigstens ein Element eines Haftverschlusses, das mit wenigstens einem kompatibel bzw. komplementär dazu ausgestalteten Element angeordnet an dem elektronischen Wirkelement in Wirkungseingriff bringbar ausgestaltet ist, so ermöglicht auch dies eine jedermann mögliche Koppelung.

Ist die Einrichtung sowohl zur Positionierung und Fixierung von wenigstens einer Elektrode, insbesondere Hautelektrode, als auch zur Positionierung und Fixierung eines elektronischen Wirkelementes ausgestattet, so ermöglicht dies, einen hohen Grad an Flexibiltät, d.h. Einsatzmöglichkeit der erfindungsgemäßen Bandage.

Sind mit der Bandage koppelbare und entkoppelbare Zuggurte vorgesehen und sind die Zuggurte wenigstens mit einer Elektrode, insbesondere Hautelektrode und/oder wenigstens einem elektronischen Wirkelement koppelbar ausgestaltet, so ermöglicht dies auch den Einsatz von Elektroden oder elektronischen Wirkelementen ohne die Bandage, d.h. z.B. während einer Autofahrt, ohne daß die Bandage die z.B. sitzende Person in nicht gewollter Art und Weise behindert bzw. einengt.

Weisen die Zuggurte eine Einrichtung zur Koppelung mit der Bandage auf und ist diese Einrichtung eine Koppelung mit der Pelotte und/oder Elektroden und/oder elektronischen Wirkelementen ermöglichend ausgestaltet, so wird dadurch mit ein und derselben Einrichtung sowohl eine Koppelung der Zuggurte mit der Bandage, als auch den zuvor genannten Dingen ermöglicht.

Weist die Einrichtung wenigstens ein Element eines mit wenigstens einem an der Pelotte und/oder wenigstens einer Elektrode und/oder elektronischen Wirkelement angeordneten Elementes eines Haftverschlusses in Wirkungseingriff bringbar auf, so wird auch dadurch auf einfachste Art und Weise eine Koppelung mit den zuvor genannten Dingen ermöglicht.

In der Zeichnung sind vier Nutzungsbeispiele der erfindungsgemäßen Bandage, insbesondere Rückenbandage, schematisch dargestellt und zwar zeigt
- Fig. 1:: die erfindungsgemäße Bandage gemäß eines ersten Nutzungsbeispieles,
- Fig. 2:: die erfindungsgemäße Bandage gemäß eines zweiten Nutzungsbeispieles,
- Fig. 3:: die erfindungsgemäße Bandage gemäß eines dritten Nutzungsbeispieles,
- Fig. 4:: die erfindungsgemäße Bandage gemäß eines vierten Nutzungsbeispieles,
- Fig. 5:: die erfindungsgemäße Bandage gemäß Fig. 4 in angelegtem Zustand in Vorderansicht und
- Fig. 6:: die erfindungsgemäße Bandage gemäß Fig. 4 in angelegtem Zustand in Rückenansicht.

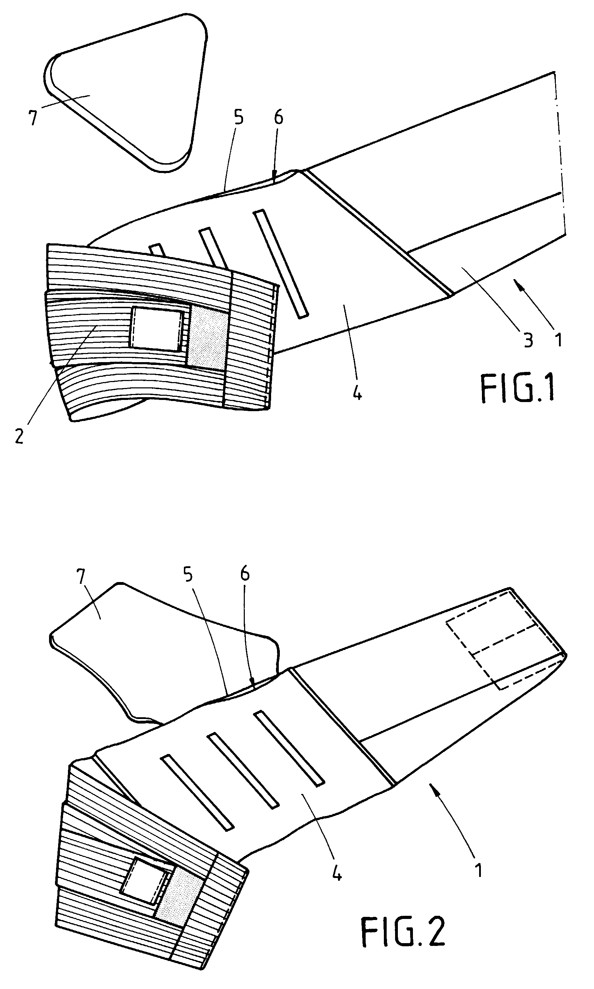

Wie der Figur 1 zu entnehmen, handelt es sich im hier vorliegenden Ausführungsbeispiel bei der Bandage um eine Rückenbandage 1, die verschiedenen Nutzungsmöglichkeiten zugeführt werden kann.

Bei der hier vorliegenden Bandage handelt es sich um eine Rückenbandage 1, d. h. Rückenorthese, mit zwei flexiblen, voneinander unabhängigen Zuggurten 2, die durch Haftverschlüsse miteinander verbunden sind.

Die erfindungsgemäße Rückenbandage 1 besteht im hier vorliegenden Ausführungsbeispiel aus einem hochfeinen Nylon/Lycragewebe und Vliesanteilen.

Durch die anatomisch gestaltete Formgebung paßt sich die erfindungsgemäße Rückenbandage 1 perfekt und individuell an jeden Patienten an.

Sie zeichnet sich des weiteren durch ein geringes Gewicht und nur geringe Bewegungseinschränkungen, selbst bei körperlicher Belastung, aus.

Die erfindungsgemäße Rückenbandage 1 ist dabei modular aufgebaut, d. h. die Zuggurte 2 sind im hier vorliegenden Ausführungsbeispiel über Haftverschlüsse, an der in Blickrichtung Fig. 1 rückgewandten Seite, an dem Bandagenkörper 3 festgelegt bzw. festlegbar, so daß die Zuggurte 2 vom Bandagenkörper 3 gelöst und auch separat getragen werden können.

Der Bandagenkörper 3 ist dabei mit einer verschließbaren Tasche 5 versehen, in der z. B. eine Sacralpelotte herausnehmbar angeordnet ist bzw. sein kann.

Wie aus Fig. 2 zu ersehen, ist die Rückenbandage 1 im hier vorliegenden Ausführungsbeispiel in ihrem mittleren Bereich 4 aus einem doppellagigen Lycragewebe derart aufgebaut, daß es zu einer Taschenbildung kommt, d. h. die Rückenbandage 1 weist in ihrem Mittelbereich 4 eine Tasche 5 auf, deren Öffnung 6 sich in Blickrichtung Fig. 2 an ihrem oberen Ende befindet.

Diese sogenannte Tasche bzw. Einschubtasche 5 ermöglicht, gemäß der Nutzungsbeispiele Fig. 1 und 2, den Einschub einer Einlegepelotte 7, wobei es sich bei dieser Einlegepelotte um eine vorgeformte Pelotte handeln kann.

Es ist dabei denkbar und hier vorgesehen, daß auch Pelotten unterschiedlicher Form und Größe aufgenommen werden können, insbesondere da in einem weiteren Ausführungsbeispiel vorgesehen ist, in der Tasche 5 ebenfalls Haftverschlüsse anzuordnen, die eine genaue Positionierung der Pelotte 7 auch bei unterschiedlicher Dimensionierung und Form ermöglichen.

Dazu ist es denkbar, die Tasche 5 selbst, d. h. die einander zugewandten Seiten, mit entsprechenden Haftverschlüssen zu versehen, so daß die Innentasche an die Form der Pelotte 7 angepaßt werden kann.

In einen weiteren Ausführungsbeispiel ist es vorgesehen, die Pelotte 7 selbst, wie z. B. eine Lumbalpelotte, Sacralpelotte oder sonstige Pelotte, mit entsprechenden Teilen eines Haftverschlusses zu versehen, so daß diese Teile mit den komplimentär dazu ausgestalteten, in der Tasche 5 angeordneten Haftverschlußteilen in Wirkungseingriff gebracht werden können, um so die, in der Tasche 5, anzuordnende Pelotte 7 zu positionieren.

Dabei ist es des weiteren denkbar, in der Tasche 5 entsprechende Haftverschlußteile für die unterschiedlichsten Pelottenformen vorzusehen.

Es ist selbstverständlich ebenso denkbar, jedwede andere Befestigungsart vorzusehen.

Als letztes ist in einem Ausführungsbeispiel vorgesehen, die Tasche 5 durch entsprechende Haftverschlüsse verschließbar und öffnungsbar auszugestalten.

Gemäß eines weiteren Ausführungsbeispieles, entsprechend Fig. 2, ist es vorgesehen, die Einlegepelotte 7 bezüglich ihrer Paßform durch Erhitzen bei z. B. 125° C individuell der jeweiligen Person anzumodellieren, d. h. unmittelbar den Bedürfnisse bzw. Formen des Patienten anzupassen.

Eine Nachformbarkeit durch punktuelles Erhitzen der Einlegepelotte 7 ist dabei selbstverständlich ebenso möglich, wobei sich die Tasche 5 und damit der Bandagenkörper 3 jeweils der entsprechenden Form anpaßt.

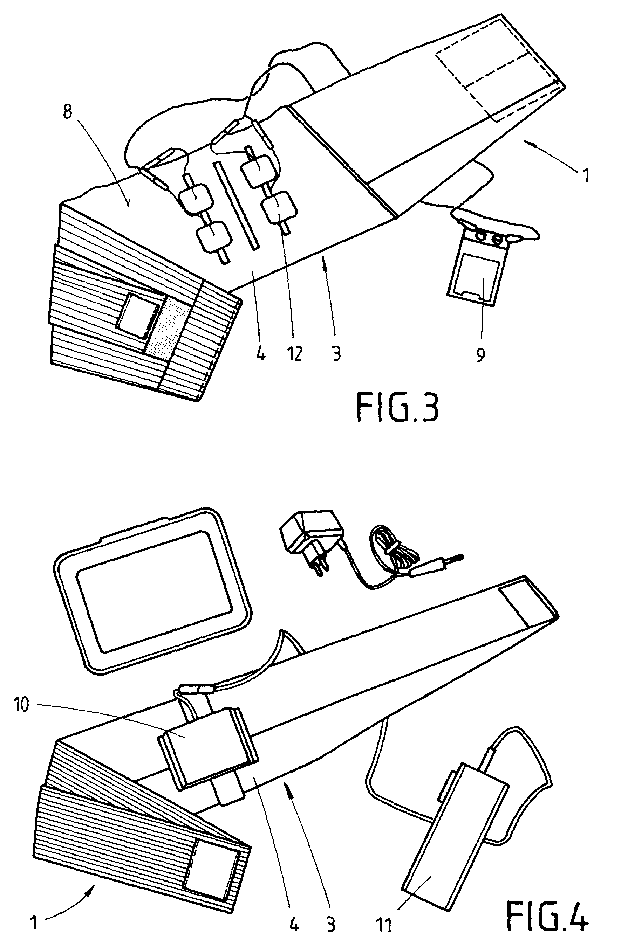

Wie der Fig. 3 zu entnehmen, ist die erfindungsgemäße Rückenbandage 1, d. h. hier insbesondere der Bandagenkörper 3, im Mittelbereich 4, d. h. auf der dem Körper zugewandten Seite 8 darüber hinaus mit entsprechenden Haftverschlüssen versehen, die eine Festlegung von Hautelektroden 12 am Bandagenkörper 3 ermöglichen, d. h. die Hautelektroden 12 sind mit den kompatibel dazu ausgestalteten Gegenstücken der Haftverschlüsse versehen, so daß die Hautelektroden 12 unmittelbar am Bandagenkörper 3, d. h. mittels der Haftverschlüsse, festzulegen sind, so daß die erfindungsgemäße Rückenbandage 1, d. h. Rückenorthese, mit einem zusätzlichen TENS-Gerät 9 gekoppelt werden kann, d. h. mit Stimulationsgeräten gekoppelt werden kann, so daß es bei Bedarf möglich ist, die Hautelektroden 12 an der Rückenbandage 1, d. h. an der der Haut zugewandten Seite 8 zu positionieren, um dann elektrische Impulse an die betroffenen, schmerzenden Zonen weitergeben zu können, um Schmerzreize zu unterdrücken bzw. vollständig beseitigen zu können.

Dabei ist es selbstverständlich möglich, die entsprechenden Haftverschlüsse an jeder gewollten Position der Rückenbandage zu positionieren oder auch bestimmte Positionen durch entsprechende Anbringung der Haftverschlußteile zu wählen.

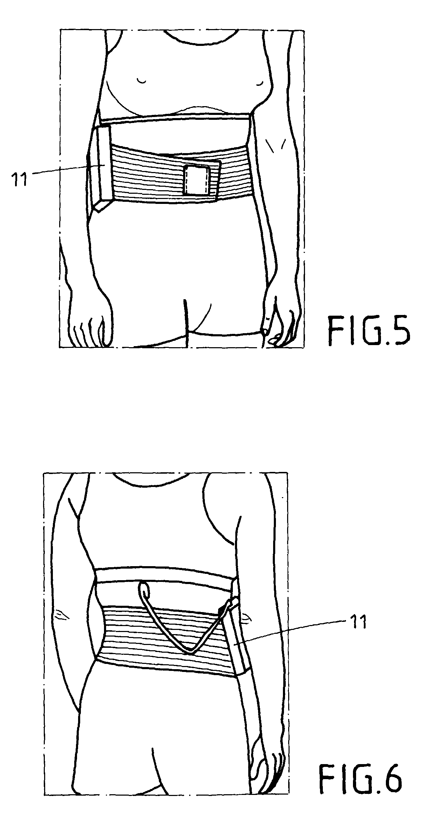

Weiterhin ist es möglich, die erfindungsgemäße Rückenbandage 1, wie der Fig. 4 zu entnehmen, mit einer Gerätschaft zur Wärmetherapie zu koppeln, d. h. auch hier sind entsprechende Haftverschlüsse, insbesondere im Mittelbereich 4, d. h. am Bandagenkörper 3 und hier auf der dem Körper zugewandten Seite 8, vorgesehen, mit deren Hilfe das elektronische Wirkelement 10, daß an der dem Körper abgewandten Seite, entsprechende kompatible Teile des Haftverschlusses aufweist, so zu positionieren ist, daß das Wirkelement an der gewünschten Stelle festgelegt werden kann, um dann z. B. über eine in der Tasche 5 befindliche Andruckpelotte auf den schmerzenden Zonen fixiert zu werden und über die elektronische Steuereinheit 11, die ebenfalls an der erfindungsgemäßen Rückenbandage 1, wie den Fig. 5 und 6 zu entnehmen, zu positionieren ist, im Schmerzzentrum wechselnde Temperaturphasen zu erzeugen.

Erfindungsgemäß ist hier eine Bandage, d. h. im vorliegenden Ausführunsbeispiel eine Rückenbandage 1, aufgezeigt, die mit den verschiedensten Gerätschaften zu bestücken ist, d. h. einem modulartigen Aufbau auweist.

Dabei ist es selbstverständlich insgesamt möglich, die verschiedenen zuvor beschriebenen Dinge gleichzeitig mit der erfindungsgemäßen Rückenbandage 1 zu koppeln oder auch nur bestimmte, d. h. die zuvor genannten Ausführungsbeispiele können insoweit gleichzeitig in den verschiedensten Kombinationen zur Anwendung gelangen.

Ebenso ist es möglich und vorgesehen, die auf der, der haut zugewandten Seite angeordneten Haftverschlußelemente sowohl zur Festlegung des elektronischen Wirkelementes als auch zur Festlegung der Hautelektroden zu nutzen. Es ist in diesem Zusammenhang möglich, die Haftelemente derart auszugestalten, daß sowohl elektronisches Wirkelement als auch die Hautelektroden an der erfindungsgemäßen Rückenbandage angeordnet werden können.

Durch die Koppelungs- und Entkoppelungsmöglichkeit der Zuggurte 2 ist darüber hinaus die Möglichkeit geschaffen, die Zuggurte 2 auch ohne den Bandagenkörper 3 tragen zu können, um dann ebenfalls durch vorgesehene, der Haut zugewandte Haftverschlüsse z. B. die Hautelektroden 12 aufnehmen bzw. tragen zu können, wobei die Elemente der Haftverschlüsse angeordnet an den Zuggurten 2 sowohl zur Festlegung an dem Bandagekörper 3 als auch zur Festlegung, z. B. der Hautelektroden 12, dienen, die an ihrer, wie bereits beschrieben, hautabgewandten Seite ebenfalls Elemente von Haftverschlüssen aufweisen.

Darüber hinaus kann selbstveständlich auch das, in Fig. 4 gezeigte elektronische Wirkelement zur Thermotherapie an den Zuggurten 2 positioniert werden, so daß diese Dinge auch bei z. B. einer sitzenden Position des zu Behandelnden, wie z. B. bei Autofahrten, zum Einsatz gelangen kann, ohne das die Anwendung der Therapie die betreffenden Personen, wie z. B. durch die Benutzung einer breiten Bandage, stört.

Gleiches gilt auch für den Einsatz von Pelotten.

Die Anordung einer derartigen Tasche und/oder derartige Tasche, wie auch Haftverschlüsse, ist selbstverständlich auch bei jedweden anderen Bandagen denkbar.

## Patentansprüche

1. Bandage, insbesondere Rückenbandage, **dadurch gekennzeichnet,** daß sie wenigstens mit einer Einrichtung (5) zur Koppelung wenigstens einer Pelotte (7) versehen ist.

2. Bandage nach Anspruch 1, dadurch gekennzeichnet, daß die Einrichtung eine Tasche (5) zur Aufnahne wenigstens einer Pelotte (7) ist.

3. Bandage nach Anspruch 1 oder 2, dadurch gekennzeichnet, daß die Tasche (5) verschließbar ausgestaltet ist.

4. Bandage nach einem der Ansprüche 1 bis 3, dadurch gekennzeichnet, daß die Tasche (5) durch wenigstens eine wenigstens teilweise vorgesehene Doppellagigkeit und wenigstens von Teilen der Bandage (1) gebildet ist.

5. Bandage nach einem der Ansprüche 1 bis 4, dadurch gekennzeichnet, daß die Tasche (5) mit Einrichtungen zur Positionierung der auf zunehmenden Pelotte (7) vorgesehen ist.

6. Bandage nach Anspruch 5, dadurch gekennzeichnet, daß die Pelotte (7) mit wenigstens einem Koppelungselement versehen ist und daß die Tasche (5) mit wenigstens einem dazu kompatibel ausgestalteten Koppelungselement versehen ist.

7. Bandage nach Anspruch 5 oder 6, dadurch gekennzeichnet, daß die Einrichtung ein Haftverschluß ist und das Tasche (5) und Pelotte (7) miteinander in Wirkungseingriff bringbare Elemente des Haftverschlusses aufweisen.

8. Bandage nach einem der Ansprüche 1 bis 7, dadurch gekennzeichnet, daß die Bandage (1) mit wenigstens einer Einrichtung zur Positionierung und Fixierung von wenigstens einer Elektrode (12), insbesondere Hautelektrode, versehen ist.

9. Bandage nach Anspruch 8, dadurch gekennzeichnet, daß die Einrichtung wenigstens ein Element eines Haftverschlusses ist, das mit wenigstens einem kompatibel dazu ausgestalteten Element angeordnet an der Elektrode (12) in Wirkungseingriff bringbar ausgestaltet ist.

10. Bandage nach einem der Ansprüche 1 bis 9, dadurch gekennzeichnet, daß eine Einrichtung zur Aufnahme einer Steuereinheit wenigstens eines Stimulierungsgerätes vorgesehen ist.

11. Bandage nach einem der Ansprüche 1 bis 10, dadurch gekennzeichnet, daß wenigstens eine Einrichtung zur Positionierung und Fixierung eines elektronischen Wirkelementes, insbesondere zur Erzeugung wechselnder Temperaturphasen vorgesehen ist.

12. Bandage nach Anspruch 11, dadurch gekennzeichnet, daß die Einrichtung wenigstens ein Element eines Haftverschlusses ist, das mit wenigstens einem kompatibel dazu ausgestalteten Element angeordnet an dem elektronischen Wirkelement in Wirkungseingriff bringbar ausgestaltet ist.

13. Bandage nach einem der Ansprüche 8 bis 12, dadurch gekennzeichnet, daß die Einrichtung sowohl zur Positionierung und Fixierung von wenigstens einer Elektrode (12), insbesondere Hautelektrode, als auch zur Positionierung und Fixierung eines elektronischen Wirkelementes (10) ausgestattet ist.

14. Bandage nach einem der Ansprüche 1 bis 13, dadurch gekennzeichnet, daß mit der Bandage koppelbar und entkoppelbare Zuggurte (2) vorgesehen sind und daß die Zuggurte (2) wenigstens mit einer Elektrode (12), insbesondere Hautelektrode und/oder einen elektronischen Wirkelement (10) koppelbar ausgestaltet sind.

15. Bandage nach Anspruch 14, dadurch gekennzeichnet, daß die Zuggurte (2) wenigstens eine Einrichtung zur Koppelung mit der Bandage (1) aufweisen und diese Einrichtung eine unmittelbare Koppelung mit wenigstens einer Pelotte (7), und/oder wenigstens einer Elektrode (12) und/oder wenigstens einem elektronischen Wirkelement (10) ermöglichend ausgestaltet ist.

16. Bandage nach Anspruch 15, dadurch gekennzeichnet, daß die Einrichtung wenistens ein Element eines mit wenigstens einem anderen an der Pelotte (7) und/oder wenigstens einer Elektrode (12) und/oder wenigstens einen elektronischen Wirkelement (10) angeordneten Element eines Haftverschlusses in Wirkungseingriff bringbar aufweist.
